Europäisches Patentamt

European Patent Office

Office européen des brevets

(11) Publication number: **0 237 666**
**A1**

(12) **EUROPEAN PATENT APPLICATION**

(21) Application number: **86302129.1**

(22) Date of filing: **21.03.86**

(51) Int. Cl.4: **C12M 1/12 , C12M 3/00**

(43) Date of publication of application:
**23.09.87 Bulletin 87/39**

(84) Designated Contracting States:
**CH DE FR GB IT LI NL SE**

(71) Applicant: **Dziewulski, David**
**2121 Burdett Avenue**
**Troy New York 12180(US)**
Applicant: **Palier, Marc S.**
**2431 21st Street**
**Troy New York 12180(US)**

(72) Inventor: **Dziewulski, David**
**2121 Burdett Avenue**
**Troy New York 12180(US)**
Inventor: **Palier, Marc S.**
**2431 21st Street**
**Troy New York 12180(US)**

(74) Representative: **Warden, John Christopher et al**
**R.G.C. Jenkins & Co. 12-15, Fetter Lane**
**London EC4A 1PL(GB)**

(54) **Membrane compartment biochemical reactor.**

(57) A biochemical reactor (10) includes a substrate compartment (12), a formation compartment (14) and a product compartment (16) connected in series with semipermeable membranes (32) between the substrate and formation compartment and between the formation and product compartment. Supply lines (36,44,46,47,60) connected to each of the compartments permits supply and circulation of a solution to all compartments. The substrate compartment further includes an influent (36) and effluent (38) line for admitting and discharging substrate solution. The substrate lines are perpendicular to each other and perpendicular to the plane of the membrane separating the substrate compartment from the formation compartment, so that a vortex can be formed in the substrate compartment to avoid fouling the membrane with solute. A magnetically coupled mixer - (50) is connected to the formation compartment for mixing the contents thereof which includes a microbe or enzyme for converting the substrate into a product.

EP 0 237 666 A1

FIG. I

## MEMBRANE COMPARTMENT BIOCHEMICAL REACTOR

### FIELD AND BACKGROUND OF THE INVENTION

The present invention relates in general to the field of microbial and enzymatic production of a useful product and, in particular to a new and useful reactor utilizing membrane separated compartments for immobilizing a catalyst used to convert a raw material or substrate into a useful product.

Recently, biocatalysis of inexpensive organic matter into valuable products other than foodstuff and alcoholic beverages, has attracted a great deal of attention. Certain desirable products are presently too expensive to synthesize chemically (for example, drugs) or are obtainable only from microbial processes - (for example vitamins and enzymes). To produce such products economically, it is important to design scientific reactors with optimal performance dynamics and biosystems.

Traditionally, stirred tank batch reactors with controlled feeding of nutrients, roller bottles or continuous flow stirred tank reactor trains with recycling have been utilized in the fermentation industry. More recently, designs have been proposed which include loop and air-lift fermenters, packed or fluidized bed reactors using biocatalyst beads, hollow fiber biochemical reactors and in an adaptation of a very old technique, solid-state fermenters, have been utilized. Modern design criteria for biochemical reactors are, continous feed, high yield and fast rate processes, robust (mixed) cultures, low technology for minimum capital investment and maintenance, on-line control for optimal operation, easy separation and concentration of continuous product streams and minimal environmental impact.

Some approaches to utilizing microorganisms to produce a product include the structure disclosed in U.S. patent 3,186,917 to Gerhard et al. A membrane is provided to pass a nutrient solution to a culture of microorganisms for producing a desired product.

U.S. patent No. 3,734,851 to Matsumura describes the use of two membranes with cells being maintained therebetween with flow of mass to and from the cells being accomplished through the respective membrane. U.S. patent No. 3,769,176 to Hise et al discloses the production of biochemicals by microorganisms and the like, wherein nutrients are supplied to the microorganisms and removed from the microorganisms by means of membranes. The following U.S. patents also describe these membranes in similar environments: U.S. patent No. 3,281,087 to Knazek et al; 3,915,802 to Kominek; and 4,241,187 to White.

### SUMMARY OF THE INVENTION

The present invention is drawn to a new continuous flow, multichamber, membrane bioreactor with built-in product separation, which overcomes many of the difficulties of the prior art and includes several advantageous features.

Accordingly, another object of the present invention is to provide a biochemical reactor for producing a product from a substrate in a substrate solution comprising, a first housing part defining a substrate compartment for receiving the substrate solution and having at least one opening, a second housing part defining a formation compartment for containing case substance to convert the substrate into the product, having at least two spaced apart openings and sealed to the first housing part. One of the two openings of the second housing part are aligned with the opening of the first housing part. The reactor includes a third housing part defining a product compartment having at least one opening aligned with the other opening of the second housing part and sealed to the second housing part. Semipermeable membranes extend across the two openings of the second housing part and separate the three compartments. An influent line is connected to the first housing part for directing substrate solution into the substrate compartment to generate a vortex of solution in the compartment. An effluent line is also connected to the first housing part for removing excess substrate from the substrate compartment. The internal circulation system (ICS) is composed of circulating lines that bypass the membranes for either filling the reactor initially or draining specific reactor compartments and is interconnected in series to each of the compartments with the formation compartment having two such lines. Mixing means are connected to the second housing part for mixing the contents thereof. Gases can be added to the formation compartment by spargers, ports and such devices in known fashion. Furthermore, gases supplied to or evolved from the second compartment are removed by means of a gas pressure relief valve (throttle) connected to the top of the second compartment.

A further object of the invention is to provide a method of producing a product from a substrate comprising supplying a solution of the substrate to a first substrate compartment having at least one semipermeable wall, permeable to the substrate, providing a formation compartment in communication with the semipermeable membrane for receiving the substrate, the formation compartment including a second semipermeable membrane for discharging a product, providing a product forming substance such as a microbe or enzyme or other catalyst in the formation compartment for treating the substrate to form the product, providing a product compartment in communication with the second semipermeable membrane for receiving the product and drawing the product from the product compartment.

A further object of the invention is to obviate or control membrane fouling.

A further object of the invention is to provide a membrane compartment biochemical reactor which is simple in design, rugged in construction and economical to manufacture.

The various features of novelty which characterize the invention are pointed out with particularity in the claims annexed to and forming a part of this disclosure. For a better understanding of the invention, its operating advantages and specific objects attained by its uses, reference is made to the accompanying drawings and descriptive matter in which preferred embodiments of the invention are illustrated.

## BRIEF DESCRIPTION OF THE DRAWINGS

In the Drawings:

Fig. 1 is an exploded perspective view of the biochemical reactor in accordance with the invention;

Fig. 2 is a side elevational view of the assembled reactor;

Fig. 3 is a sectional view taken along line 3-3 of Fig. 2 showing the substrate compartment where raw materials to be processed are supplied;

Figs. 4a, 4b and 4c are respective sectional views taken along lines 4a-4a, 4b-4b, and 4c-4c, showing a formation compartment where microbes or enzymes act on the substrate or raw material to produce a product;

Fig. 5 is a sectional view taken along line 5-5 of Fig. 2 showing a product compartment where products formed in the formation compartment are collected and withdrawn from the reactor;

Figs. 6a and 6b are side elevational and top views respective of reactors in accordance with other embodiments of the invention where plural formation processes may take place;

Fig. 7 is a schematic representation of the reactor in Fig. 2 showing lines for supply and removal of various substances;

Fig. 8 is a graph showing various concentrations and characteristics of substances in the compartments plotted against time for an experiment utilizing the reactor;

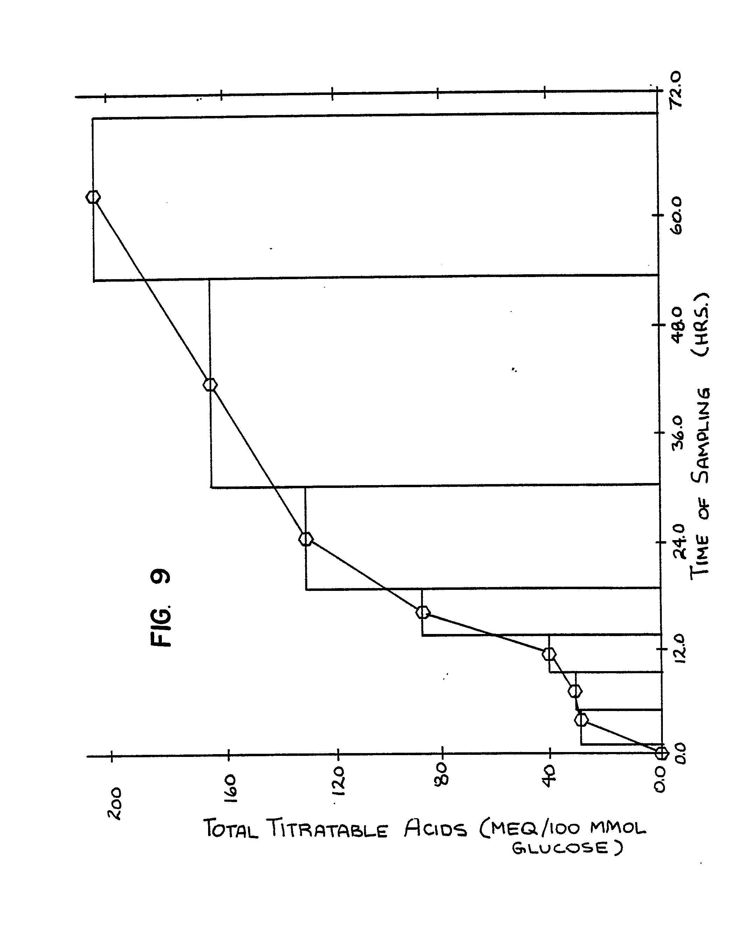

Fig. 9 is a graph showing the amount of titratable acids plotted against time for the experiment;

Fig. 10 shows an alternate form of the mixer for the reaction compartment;

Fig. 11 is an alternate form of the membrane arrangement; and

Fig. 12 shows the use of paddles to keep one of the membranes free of caking.

## DESCRIPTION OF THE PREFERRED EMBODIMENTS

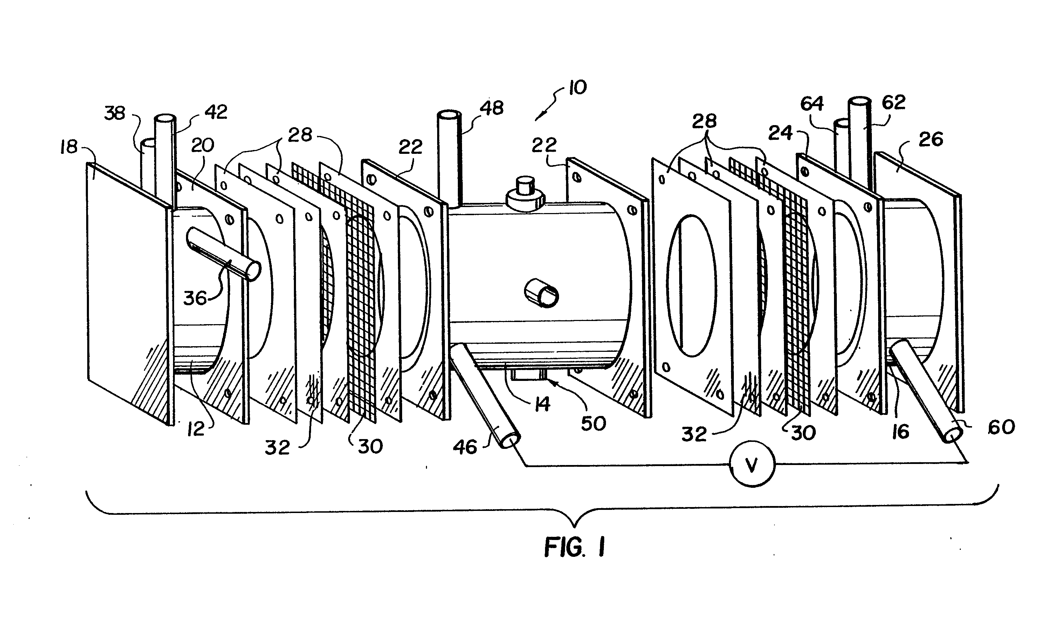

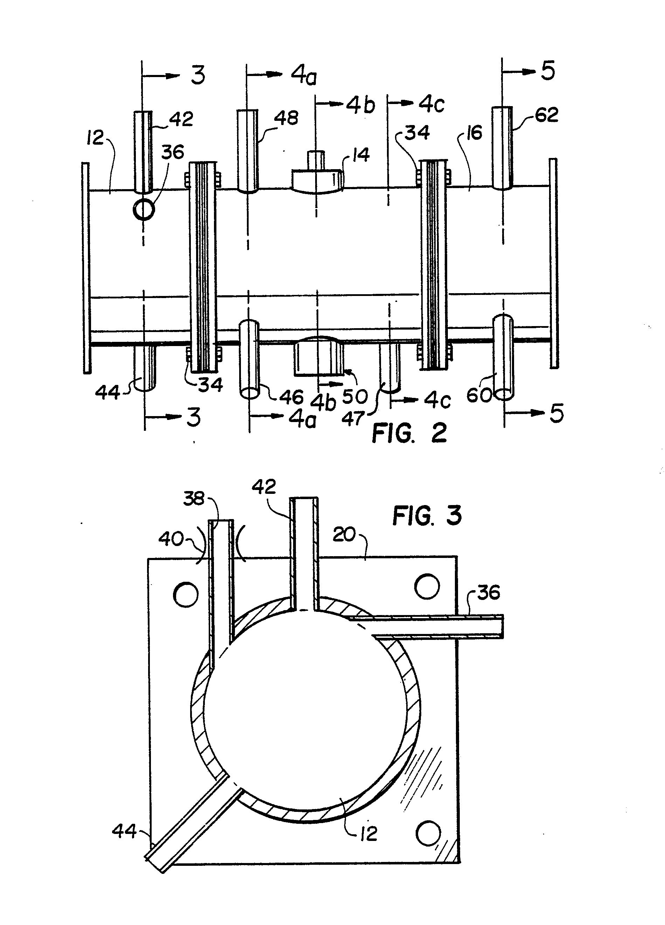

Referring to the drawings in particular, the invention embodied therein, in Figs. 1 and 2, comprises a membrane compartment biochemical reactor, abbreviated MCBR, generally designated 10. The reactor includes three separate compartments formed of cylinders made of ¼ inch thick steel and welded plates all of which are covered inside and out with TEFLON (a registered trademark of the DuPont Company for tetrafluoroethylene polymers and the like). Steel pipe stand-offs also covered inside and outside with TEFLON are connected to openings in the cylinders by brazing.

A substrate delivery compartment 12 includes end plates 18 and 20. Plate 20 has a circular opening therein which aligns with circular openings in end plates 22 of a formation compartment 14. A product compartment 16 includes end plates 24 and 26, with end plates 24 including a circular opening aligning with the openings of plates and 22.

A membrane packet is provided between plates 20 and 22, and between plates 22 and 24. Each packet comprises three gaskets 28 made of suitably inert material which is also capable of forming a seal (such as silicone or TEFLON), a supporting screen 30 and a semipermeable membrane 32. Gaskets 28 all have circular openings therein aligned with the openings of plates 20, 22 and 24. The plates are connected by bolts 34, in known fashion, to produce pressure seals between the three compartments.

As best shown in Fig. 3, the substrate delivery compartment is supplied with substrate solution containing a raw material that is to be processed, over an influent line or pipe 36. The substrate is pumped into chamber 12, under pressure and tangentially to the cylinder to establish a vortex in the chamber 12 which is perpendicular to the membrane surface. Substrate is removed from chamber 12 over effluent line or pipe 38, which is perpendicular to line 36 to establish a pressure gradient across the lines and pressurized compartment 14. Effluent substrate from pipe 38 is returned to the substrate reservoir. A throttle schematically shown at 40 may also be provided, in the form of packing or valve or the like, to reinforce this pressure gradient. By controlling the applied pressure to compartment 12 the amount of flux through the membrane 32 into compartment 14 can be regulated. The length of compartment 14 coupled with the pressure induced flux determines the residence time through the formation compartment 14.

The internal condition of compartment 12 is monitored over a pressure monitor line 42. Compartment 12 is equipped with one internal circulation line 44.

By establishing a vortex in chamber 12, solute deposition on the membrane is prevented. The pressure gradient is the primary driving force (convective transport) for the medium's movement through the entire reactor along with diffusive transport as a secondary driving force.

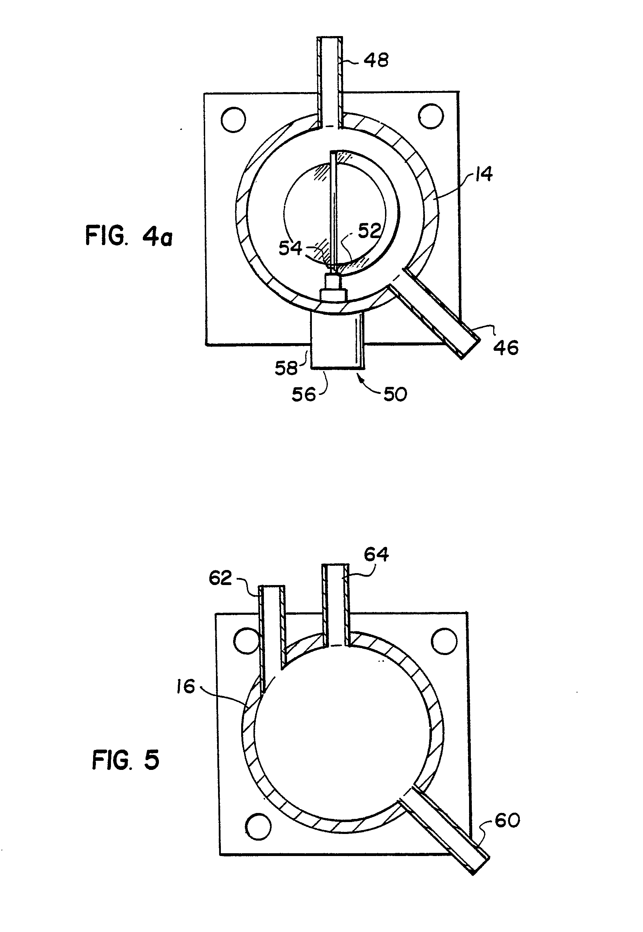

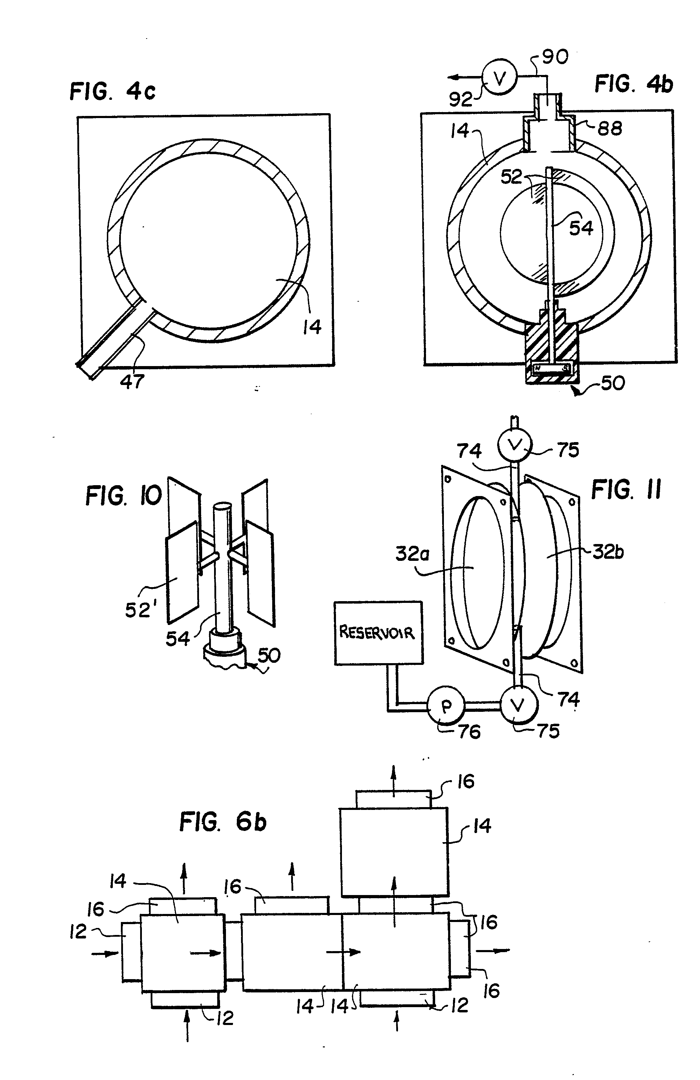

Turning to Figs. 4a, 4b and 4c, formation chamber 14 is provided with cells, enzymes or a suitable catalyst for converting or reacting with at least one component of the substrate solution to produce a useful product.

Chamber 14 is equipped with two internal circulation system lines 46 and a pressure monitoring line 48.

In addition, chamber 14 includes mixing means generally designated 50 in the form of a mixing paddle 52 connected to a shaft 54 which is in turn connected to a magnet 56 for magnetic rotation of paddle 52. Mixing means 50 is hermetically sealed in a casing 58 to avoid any possible contamination to the interior of compartment or chamber 14. For this purpose, means 50 is completely fabricated of TEFLON. Furthermore, compartment 14 includes a means of removing gases supplied to or evolved from this compartment.

As shown in Fig. 5, a product compartment or chamber 16, in communication with formation chamber 14 over one of the semi-permeable membranes 32, includes an internal circulation system line 60, a pressure monitoring line 64 and a product stream effluent line 62.

Compartment 14 acts as a continuously-stirred-tank reactor (CSTR) while compartments 12 and 16 input and output material respectively, as a plug. This reactor acts as a combined plug-flow immobilized reactor CSTR. Materials making up the reactor are specifically chosen for their resistance to adverse conditions, for example, autoclaving and ultraviolet radiation.

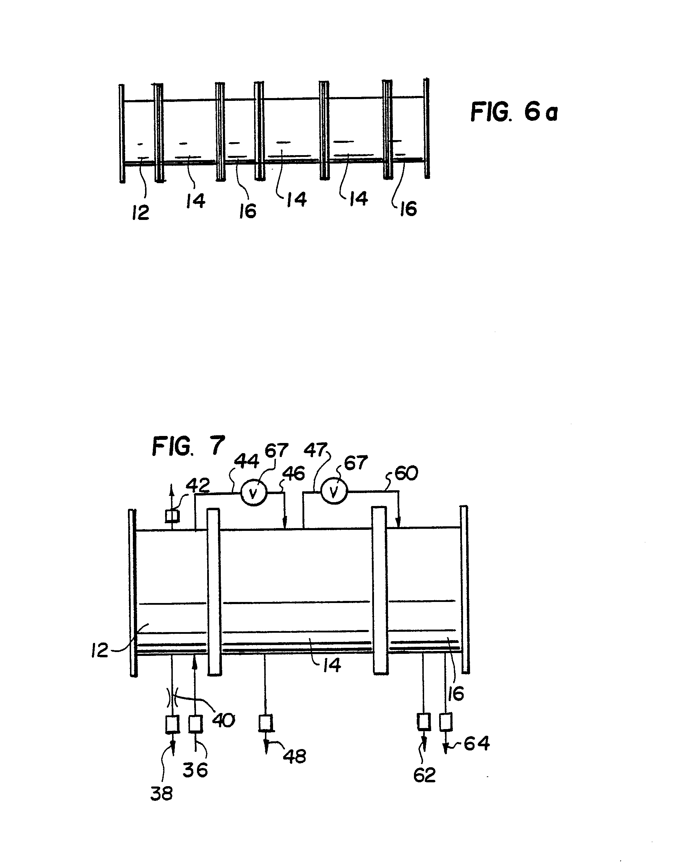

As shown in Figs. 6a and 6b, where corresponding numerals are utilized to designate corresponding parts, a plurality of formation compartments 14 may be provided in series, with or without intermediate product chambers 16, to effect serial formation of a product which requires more than one formation step; for separating/concentrating a product; and for means of altering the fluid exiting one compartment 14 before entering another compartment 14. An enzyme produced product in the first formation chamber 14 can thus be processed in the first product chamber by having an additional component added thereto, and thereafter be supplied to a second formation chamber 14 for further processing. Two formation chambers 14 may be provided one after the other with different microbes, or enzymes or suitable catalyst therein for modifying a product produced in the first formation compartment, further in the second formation compartment.

The circuit of the inventive reactor is shown schematically in Fig. 7 where again the same reference numerals designate similar parts.

In operation, the inventive reactor is first filled with the substrate solution through 36 where it is then transported via the internal circulation system lines 44, 46, 47 and 60 to the other compartments. Once the reactor is completely filled, these lines are then closed off by valves 67 and peripheral equipment such as pressure monitors (not shown) connected to lines 42, 48 and 62 are turned on. Substrate solution is then continuously circulated through line 36 into compartment 12 and out through line 38 which is throttled to induce a pressure in compartment 12. Therefore, a controlled flux of substrate continuously enters through the left hand membrane 32 (as shown in Fig. 1) into the center of formation compartment 14 of the reactor. The microorganisms contained in the center compartment 14, which are rapidly mixed with the incoming substrate by mixer 50, grow until some desired state is reached. The microorganisms generate the desirable products which flow with solvent into the product compartment 16 over the right hand membrane 32.

As noted above, the reactor can be expanded due to its modular structure with special membranes being utilized with several cultures or enzymes to produce useful products in multiple steps.

Further, product concentration can be achieved by various separation techniques. Membrane fouling can be either obviated or controlled by one or more of the following methods:

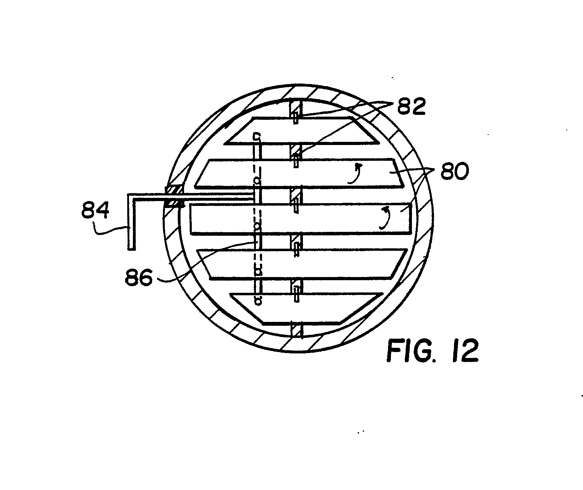

(1) Use of a vertical paddle (impeller) in place of blades to increase the shear force induced on the membrane surface. Depending upon the Reynolds number of the impeller 52' this shear stress will cause some equilibrium thickness of cake (as shown in Fig. 10.

(2) Pulsation and relaxation of the membrane by a series of increases and decreases of the applied pressure generated in compartment 12.

(3) By placing two membranes 32a, 32b as shown in Fig. 11, in place of the single membrane 32 in the assembly between chambers 14 and 16 and placing appropriately sized tubes 74 between the membranes at the bottom and top margins and then assembling the reactor, a slit (small space) exists between the membranes. Attached to the tubes at the bottom and top are valves 75 (throttles). In addition a pump 76 is placed before the valve at the bottom of the reactor. This pump is connected to a reservoir containing an appropriate sterile solution such as saline or balanced salt solution. When membrane fouling occurs, the valve at the bottom is opened and pumping is initiated. The space (slit) between the membranes is filled with fluid which, due to the resistance of the closed upper valve, is expressed across both membranes into chambers 14 and 16, thus removing the deposition layer (cake) of the membrane facing compartment 14 by backwashing. Once fluid is expressed (actually a short pulse) through the membranes, the valve at the top is opened to relieve the pressure and to prevent membrane bursting. The pump is shut off simultaneously with the closing of the upper valve, after which the lower valve is closed and operation continues as before. This procedure can be performed without interruption of reactor operation and can be repeated as necessary (or until the desired flux is re-attained).

(4) Use of one or more paddles 80 located very close to membrane 32 as shown in Fig. 12, leading to compartment 16, that is very rapdily moved back and forth across the front of this membrane, without making contact, to create local high shear stresses on the membrane that breaks up the cake. The paddle is only used when the cake thickness on the membrane reduces the flux through the membrane below an acceptable level. Each paddle is hinged at 82 to a fixed support in the compartment and pivoted by a liner 84 over a common tie rod 86.

These methods of cake reduction can be used either in concert with each other or separately in both the singular and plural form of the MCBR.

As shown in Fig. 4b, a vapor or bubble trap 88 is provided at the top of compartment 14 with a gas line 90. Valve 92 may be provided in line 90 to automatically vent gas under pressure in the trap. Gas may also be supplied to compartment 14 (e.g. over line 36 and membrane 32) if needed by the organisms or reactions in the compartment, and the gas can be vented over line 90. Alternative mixing or agitation devices can also be installed to replace the magnetically coupled stirring or mixing means 50.

Experiments, one of which will be set forth in detail hereinunder, demonstrate that the processes which could be developed utilizing the performance potential of the inventive reactor, for generating valuable products, would be more economical and have higher productivity than current processes now being employed. Such products include organic intermediates biochemicals, enzymes and biomass.

The savings and increased productivity are a result of unique characteristics and capacities of the reactor, as follows: continuous operation; clear, sterile effluent; low energy input requirements; low initial capital and maintenance costs; possibility of separate multi-culture fermentation, enzymatic alteration of products, and/or product steam separation and concentration; ability to rapidly bring culture to, and control culture during, maintenance phase; immobilized fed-batch operation; ease of reactor control; production of large biomass from low substrate concentrations; simultaneous broth agitation and controlled gas transfer when needed; sterilizability; and the modular nature of the reactor. An experiment utilizing the invention will now be set forth in detail.

Before the experimental results obtained from tests runs with the membrane biochemical reactor are discussed, the experimental conditions and analytical techniques used, are outlined.

E. coli C3000 was held in stock on nutrient agar slants at 4°C. Two days prior to a run, the culture was transferred from the slant to a Davis salt solution containing 0.2% glucose, and incubated for 24 hours at 37°C. One millimeter of the resulting culture was then transferred to fresh davis media and incubated for 12 hours at 37°C. an additional transfer of 1 ml was made from the 12 hour culture to 99 ml of fresh Davis media. After 6 hours of incubation at 37°C, a 1 ml aliquot was injected into the center compartment 14 of the reactor 10.

The apparatus was prepared for an experimental run by coating all interior and exterior surfaces of the reactor three times with Fluro-Glide FB (Chemplast, Inc., Wayne, New Jersey). Membranes 32 (which is made of polypropylene micro-porous material for example) and screens 30 were mounted, and the reactor was assembled. A reservoir (not shown) containing 2 liters of 2X basal salts was attached to the reactor, and all open ports (of the various lines) were packed with fiberglass wool and capped with aluminum foil. The assembly was then autoclaved at 121°C and 15 psig for 40 minutes. After sterilization, the reactor was immediately removed and transported to a laminar flow hood (model B40-112 BioGard Hood, the Baker Co., Inc. Sanford, Me. and not shown) where all ports capped for sterilization were fitted with 0.22 $\mu$ microporous filters 66 in Fig. 7 (FPO30/3, Schleicher and Schuell, Inc. Keene, NH). Two and one-half liters of 0.4% glucose were prepared and autoclaved at 121°C and 15 psig for 30 minutes. While still hot, 2 liter of the glucose were added to the reservoir in the laminar flow hood. Reactor integrity was then checked, after which the assembly was placed into a 37°C incubator, the entire set-up was UV sterilized for 15 minutes, and then allowed to equilibrate overnight. The following day, a feed line to the substrate solution influent stand-off 36 was connected to a peristaltic pump and pressure tested for 1 hour at 3 psig with mixing in the center compartment 14.

After successfully pressure testing the reactor, cannulas of 4 and 3 inches were aseptically placed through septa into sampling ports of compartment 14 and 16 respectively as well as the effluent substrate line 38.

The left and right hand compartments 14 and 16 were sampled regularly for pH, and glucose concentration. The center compartment 14 was initially sampled for pH, and turbidity. Thirty minutes after seeding, and concomitantly, thereafter, with other compartments a 1 ml sample from the center compartment 14 was withdrawn through the sampling port for viable counts and turbidity. Prior to sampling, membrane flux was measured and incubator temperature was checked. After sampling, the interior of the incubator was decontaminated via UV for 7 to 10 minutes.

The analytical methods used for viable counts, turbidity, volume flux, and glucose concentration as well as total titratable acids were as follows:

(1) Samples (1 ml) withdrawn from the center compartment 14 were immediately diluted in Davis salt. Nutrient agar spread plates of each dilution were then prepared in duplicate and incubated at 37°C for 12 hours before counting.

(2) A portion of the raw sample from the viable counts was placed into a 0.5 ml micro-cuvette and read at 620 nm with a Model 552 Spectrophotometer (Perkin-Elmer, Norwalk, Ct.)

(3) Prior to sampling, the product line (64) was closed and the amount of time required for a manometer connected to line 62 to register a one-millimeter change was recorded in seconds.

(4) A modified (Fisher Scientific) method was used to determine the glucose concentration of the solution taken from the substrate effluent line 38 and the sampling part of compartment 16.

(5) Product samples (approx. 200 ml) were collected at regular intervals from the sampling port of compartment 16 and refrigerated at 4°C until total acids could be titrated. A pH meter was used to determine a pH 7.1 end-point.

The experimental results were as follows:

To determine the general growth characteristic of the model organisms (E. coli C3000), first a batch growth curve was run. E. coli stock was prepared as outlined previously. Ten 250-ml Erlenmeyer flasks containing 99 ml of sterile Davis minimal media (0.2% glucose) were incubated at 37°C until equilibrated with incubator temperature. Proposed stock was then seeded at a rate of $1.0^{P'}$ ml per flask. Each flask in the series was used for 3 or 4 sequential samplings. The time interval between samples was determined by the turbidity of the previous sample. Once the sequential samples were taken, the flask was discarded and the next flask in the series was used. Therefore, only 3 or 4% of the total culture volume was removed from any one flask. At each sample point a coincident turbidity measurement was made. The calculated specific growth rate for log-phase ($\mu_{log}$) was 1.16/hr.

The following conditions and procedures were typical for a test with the inventive reactor using E. coli C3000:

$t_c$ = 77 min (delay before continuous operation)

$V_c$ = 823 ml (one center chamber 14 volume)

$T$ = 37°C (controlled temperature)

$P$ = 3 psig (inlet chamber 12 pressure)

$\omega$ = 640 rpm (speed for magnetically coupled stirrer 50)

$J_{vo}$ = 0.49 x 10$^{-1}$ cm/min (initial flux in chamber

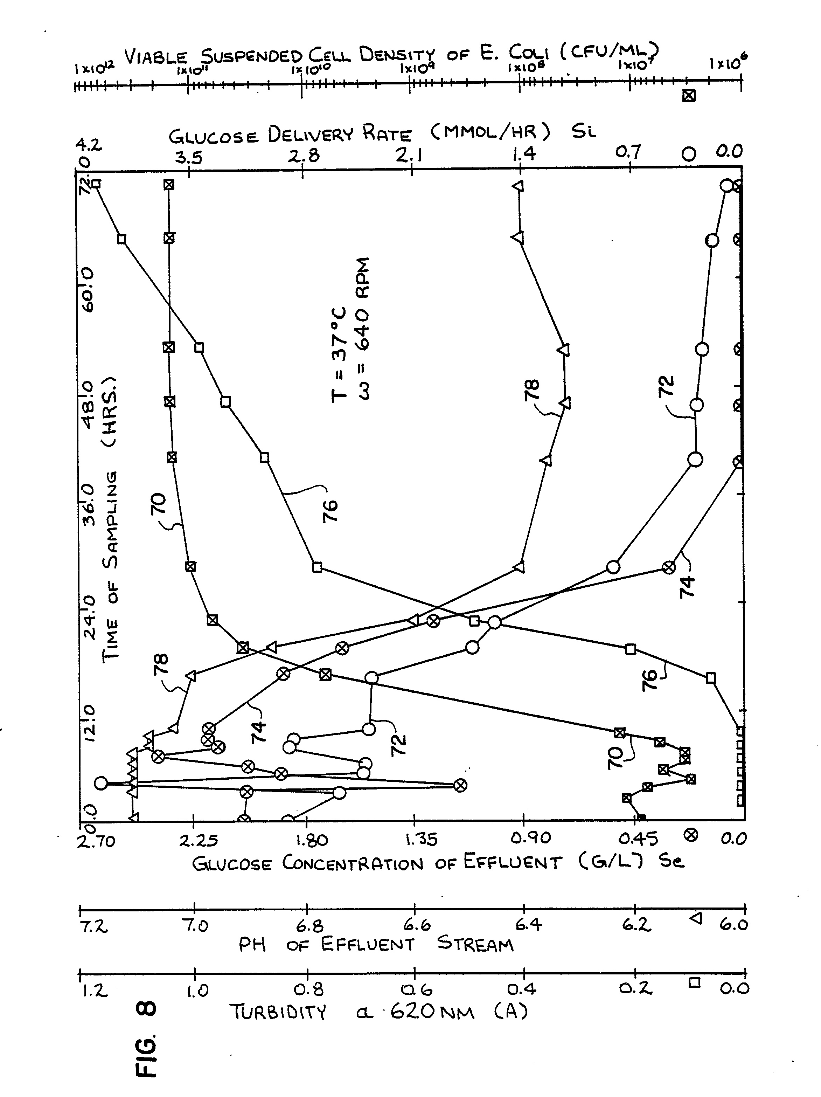

One milliliter of 1.11 x 10$^{11}$ cfu/ml culture was inoculated into the center compartment 14 (823 ml total volume) at time t=t$_o$. The dilution effect after mixing should have resulted in a cell count of 1.35 x 10$^8$ cfu/ml. However, at the 0.5-hour mark (see Fig. 8 curve 70) during reactor operation, a sample withdrawn from the center compartment 14 resulted in a viable count of 8.5 x 10$^5$ cfu/ml. The apparent loss in cell number was attributed to culture transport to the effluent or product membrane 32 on the right in Fig. 1, and hence the beginnings of cake formation on that membrane. The cell mass may be divided into two fractions: suspended call mass ($X_S$) and the cell mass involved in the case ($X_c$), where,

$X_t = X_s + X_c$

and $X_t$ is the total cell mass contained in the center compartment 14.

A typical bacterial growth curve 70 can be seen in Fig. 8. The specific growth rate for the log-phase - ($\mu_{log}$) was 0.8934/hr and for the stationary phase ($\mu_{sta}$) was approximately equal to zero.

Fig. 8 also shows the flux decline associated with cell growth as curve 70 levels off. As $X_c$ increases, there is a corresponding decrease in flux ($J_v$) due to the cake formation by the bacteria transported to the membrane.

The glucose concentration was monitored in the left and right compartments 12 and 16 (influent and effluent or product respectively) and the results are presented in Fig. 8 at curves 72 and 74. At $t = 30$ hours the influent flux decreased, possibly assisting in the onset of the stationary maintenance phase. The effluent glucose concentration ($S_e$) follows the familiar trend of reduction related to increasing culture density. After $t = 40$ hours the effluent stream (curve 74) is free of glucose.

The effect of flux on the amount of glucose that was delivered to the microorganisms is apparent in curve 72. The glucose delivery rate (GDR, where $J_v A_o {}^* S_i$ = GDR, and A is the membrane surface area) decreased with increasing microbial numbers. It should be noted that the decline in GDR is due to the decline in $J_v$ as a result of cake formation on the effluent membrane (right membrane 32).

Ultimately, the bacterial growth will itself limit the GDR. This coupling of transport and biological parameters should lead to a prolonged maintenance phase; eventually, an equilibrium of cake thickness, GDR, and culture should occur. At this point the effluent stream should not contain any substrate, as seen in Fig. 8 beginning at T = 40 hours. Rather than depending upon the natural occurrence of equilibrium, cake formation can also be mechanically controlled at any point in time by one or more of the methods mentioned earlier.

The rate of change of turbidity (curve 76) decreased (remained linear), but did not level off completely. It is difficult to say whether the beginnings of a maintenance phase was indicated or if simple stationary phase was attained. The results of prolonged runs (180 hours) should determine if maintenance phase was actually achieved.

Since E. coli (and the Enterobacteriaceae in general) produces products common to a mixed acid fermentation, the effluent stream in preliminary runs was monitored for products by pH. Fig. 8 at curve 78 shows that at $t = 30$ hr the effluent or product sampled from compartment 16 had a pH of 6.4 and remained steady until termination of the experiment (an additional 42 hours) while the influent pH was at 7.1. This obviated the need for addition of NaOH. Twelve hours after the effluent pH reached 6.4, no glucose was evident in the effluent sample (curve 72). This indicated that total glucose turnover was occurring and that the culture may have reached a stationary/maintenance phase.

It should also be noted that from the onset of continuous operation, the effluent or product stream (at line 64) for the entire length of the run remained clear of any turbidity. This effluent was collected aseptically in a sterile 1-liter erlenmeyer flask fitted with a filling bell. At regular intervals, effluent samples - (approx. 200 ml) were taken from the bulk effluent and stored at 4°C until total titratable acids could be determined. After the sample was taken, the remainder of the bulk was discarded, and a new sterile 1-liter flask was used for collection. The samples were processed two weeks after termination of this experiment and the results were presented in the Table.

After the termination of a run, the reactor is drained and the interior is examined. This particular experiment showed that the TEFLON coat remained intact since no rust was observed. Also, the effluent membrane had a very thick (~2mm), gel-like bacterial cake covering the entire surface, as anticipated. Cake formation (i.e. membrane fouling can be controlled by various methods mentioned above.

## TABLE

| | |
|---|---|
| Total Viable Cell Number | $1.42 \times 10^{14}$ CFU/l |
| Total Equivalent Cell Mass (*) | 41.5 g/l |
| $\mu_{log}$ | $0.8934 \ hr^{-1}$ |
| $\mu_{sta}$ | $\approx 0$ |
| Influent Stream, pH | 7.1 |
| Influent Stream, Glucose Concentration | 2.0 g/l (0.20%) Glucose in DAVIS Salts |
| Effluent Stream | Clear & Sterile |
| Effluent Stream, pH | 6.4 at t = 70 hrs |
| Effluent Stream, Average Total Titratable Acids (+) | |
| t=1-5 hrs | 30.0 meq/100 mmol Glucose |
| t=5-9 hrs | 31.6 meq/100 mmol Glucose |
| t=9-13 hrs | 40.1 meq/100 mmol Glucose |
| t=13-18 hrs | 87.2 meq/100 mmol Glucose |
| t=18-29 hrs | 131.4 meq/100 mmol Glucose |
| t=29-52 hrs | 165.7 meq/100 mmol Glucose |
| t=52-70 hrs | 204.5 meq/100 mmol Glucose |
| Total Volume Through Reactor | 7.6 Reactor Volumes over 70 h ($\sim$40% passed through reactor during production of 100 meq/ mmol Glucose or more). |

*$3.42 \times 10^{9}$ cells/mg for E. coli (Palmer Malette, J. Gen. Physiol. 45,229 (1961)

+ Samples were refrigerated, and analyzed two weeks later.

While specific embodiments of the invention have been shown and described in detail to illustrate the application of the principles of the invention, it will be understood that the invention may be embodied otherwise without departing from such principles.

**Claims**

1. A biochemical reactor for producing a product from a substrate in a substrate solution comprising:
a first housing part defining a substrate compartment for receiving a substrate solution and having at least one opening:
a second housing part defining a formation compartment for containing at least one of cells, enzymes and catalyst that converts a substrate into a product, the formation compartment having at least two spaced apart openings and being sealed to said first housing part, said one opening of said first housing part aligned with one of said at least two openings of said second housing part;

a third housing part defining a product compartment for receiving a product formed in said formation compartment and having at least one opening, said third housing part sealed to said second housing part with said opening of said third housing part aligned with the other of said at least two openings of said second housing part;

a semipermeable membrane extending across each of said at least two openings of said second housing part and separating said formation compartment from said substrate and product compartments respectively;

stirring means associated with said second housing part for stirring contents of said formation compartment;

gas removal means connected to said second housing part for venting gas from said formation compartment;

an influent substrate solution line connected to said first housing part and disposed with respect to at least one of said membranes so as to generate a moving vortex of solution in said substrate compartment to avoid fouling of said at least one membrane;

an effluent substrate solution line connected to said first housing part extending at an angle to said influent line, for discharging solution from said substrate compartment; and

at least one circulation line connected to each of said first and third housing parts and at least two circulation lines connected to said second housing part for supplying and withdrawing fluid to and from each of said substrate, formation and product compartments respectively.

2. A reactor according to claim 1, including a support screen extending across each of said at least two openings of said second housing part for supporting a respective one of said membranes.

3. A reactor according to claim 2, wherein each of said housing parts is cylindrical, said first housing part including a pair of end flanges, one of said end flanges carrying said at least one opening, said second housing part having a pair of end flanges each carrying one of said at least two openings, one of said flanges of said second housing part connected to said flange of said first housing part which carries said at least one opening, said third housing part including opposite flanges one of which carries said at least one opening of said third housing part, said at least one flange of said third housing part connected to the other of said flanges of said second housing part.

4. A reactor according to claim 3, wherein each membrane and screen is separated by gaskets and connected between one flange of said second housing part and a flange of one of said first and third housing parts.

5. A reactor according to claim 3, wherein said influent substrate solution line is substantially perpendicular to said effluent substrate solution line, said influent substrate solution line extending tangentially to said cylindrical first housing part.

6. A reactor according to claim 3, wherein each of said circulation lines is connected at a lower portion of each of said first, second, third housing parts respectively.

7. A reactor according to claim 1, wherein said reactor is made of steel with a non-rusting sterilizable surface coating thereon.

8. A reactor according to claim 1, wherein said influent substrate solution line extends at an angle to said effluent substrate solution line and said influent substrate solution line is directed tangentially to a surface of said first housing part substrate compartment and said formation compartment, with a throttle in said effluent substrate solution line for establishing an overpressure in said substrate compartment.

9. A reactor according to claim 1, wherein said stirring means comprises a paddle rotatably mounted in said formation compartment, a shaft extending through said second housing part, a magnet connected to said shaft and a housing sealing said magnet and shaft whereby said magnet is activatable externally for rotating said paddle.

10. A method of producing a product from a substrate in a reactor having a substrate compartment, a formation compartment and a product compartment with a first semipermeable membrane separating the substrate compartment from the formation compartment and a second semipermeable membrane separating the formation compartment from the product compartment, comprising:

supplying a solution of substrate under pressure to the substrate compartment so that substrate passes through the first semipermeable membrane;

providing at least one of cells, enzymes and a catalyst in the formation compartment which acts upon the substrate to form the product, the substrate solution being provided under sufficient pressure to cause the product to move through the second semipermeable membrane into the product compartment; and

removing the product from the product compartment.

11. A method according to claim 10, including supplying the substrate solution to the substrate compartment tangentially to a surface of the first semipermeable membrane to avoid deposition of a solute from the solution on the first semipermeable membrane.

12. A method according to claim 11, including stirring the contents of the formation compartment.

13. A method according to claim 12, including discharging an amount of substrate solution from the substrate compartment at a rate to establish an overpressure in the substrate compartment above a pressure in the formation compartment and the product compartment.

14. A method according to claim 10, including removing gas from the formation compartment.

15. A method according to claim 14, including adding a gas to the formation compartment which is then used in production of the product.

16. A method according to claim 10, including agitating contents of the formation compartment adjacent the second permeable membrane to reduce the formation of the cake of material on the second semi-permeable membrane.

17. A biochemical reactor for producing a product from a substrate in a substrate solution comprising:

a first housing part defining a substrate compartment for receiving a substrate solution and having at least one opening;

a second housing part defining a formation compartment for containing at least one of cells, enzymes and catalyst that converts a substrate into a product, the formation compartment having at least two spaced apart openings and being sealed to said first housing part, said one opening of said first housing part aligned with one of said at least two openings of said second housing part;

a third housing part defining a product compartment for receiving a product formed in said formation compartment and having at least one opening, said third housing part sealed to said second housing part with said opening of said third housing part aligned with the other of said at least two openings of said second housing part;

a semi-permeable membrane extending across each of said at least two openings of said second housing part and separating said formation compartment from said substrate and product compartments respectively; and

means for supplying the substrate to said first housing part under pressure greater than the pressure of the substrate in said second housing part so that the substrate passes through said semipermeable membrane extending across the opening of said second housing part separating said substrate compartment from said formation compartment.

18. A reactor according to claim 17, including stirring means associated with said second housing part for stirring contents of said formation compartment.

19. A reactor according to claim 17, including gas removal means connected to said second housing part for venting gas from said formation compartment.

20. A reactor according to claim 17, including an influent substrate solution line connected to said first housing part and disposed with respect to at least one of said membranes so as to generate a moving vortex of solution in said substrate compartment to avoid fouling of said at least one membrane.

21. A reactor according to claim 20, including an effluent substrate solution line connected to said first housing part extending at an angle to said influent line, for discharging solution from said substrate compartment.

22. A reactor according to claim 17, including at least one circulation line connected to each of said first and third housing parts and at least two circulation lines connected to said second housing part for supplying and withdrawing fluid to and from each of said substrate, formation and product compartments respectively.

FIG. 1

0 237 666

FIG. 2

FIG. 3

FIG. 4a

48

14

52

54

58 56 50 46

FIG. 5

62 64

16

60

FIG. 4c

FIG. 4b

47

14

90

92

14

88

52

54

50

FIG. 10

52'

54

50

FIG. 11

74

75

32a

32b

RESERVOIR

76

75

74

P

V

16

14

FIG. 6b

14

16

16

12

16

12

14

14

12

16

FIG. 6 a

FIG. 7

FIG. 8

FIG. 9

0 237 666

FIG. 12

European Patent Office

**EUROPEAN SEARCH REPORT**

Application number

EP 86 30 2129

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X,Y | EP-A-0 155 237 (MBR BIO REACTOR AG)<br><br>* Claims 1-3,9; page 3, lines 5-10,19-23; page 5, lines 3-11; page 7, lines 25-30; page 8, line 30 - page 9, line 21; figures 1,6-9 *<br><br>--- | 1,2,4, 5,10, 11,13- 15,17, 19-21 | C 12 M 1/12<br>C 12 M 3/00 |
| Y | US-A-4 204 962 (G.A. FORD)<br><br>* Figure 8; column 9, lines 33-39 *<br><br>--- | 1,5,11 ,20,21 | |
| A | DE-A-1 959 609 (INTERMAG)<br><br>* Claim 1 *<br><br>--- | 10,13, 17 | TECHNICAL FIELDS SEARCHED (Int. Cl.4) |
| A | WO-A-8 401 959 (UNIVERSITY OF CALIFORNIA)<br>* Abstract; figure 1 *<br><br>--- | 1,10 | C 12 M |
| A | FR-A-2 393 849 (BATTELLE-INST.)<br>* Claims 1-3; figures 1,2 *<br><br>----- | 1,10 | |

The present search report has been drawn up for all claims

| Place of search | Date of completion of the search | Examiner |
|---|---|---|
| THE HAGUE | 07-11-1986 | WIESER, M.R.J. |